# EUROPÄISCHE PATENTSCHRIFT

(11) **EP 1 324 793 B1**
(45) Veröffentlichungstag und Bekanntmachung des Hinweises auf die Patenterteilung: **22.12.2004**
(21) Anmeldenummer: 01951309.2
(22) Anmeldetag: 31.07.2001
(51) Int. Cl.: A61M 5/315

(54) **VORRICHTUNG ZUR VERABREICHUNG EINES INJIZIERBAREN PRODUKTS**
DEVICE FOR ADMINISTERING AN INJECTABLE PRODUCT
DISPOSITIF POUR L'ADMINISTRATION D'UN PRODUIT INJECTABLE

(30) Priorität: 26.09.2000 DE 10047637
(43) Veröffentlichungstag der Anmeldung: 09.07.2003
(73) Patentinhaber: TecPharma Licensing AG, 3401 Burgdorf (CH)
(72) Erfinder: GATTI, Stefan, CH-3415 Hasle-Rueegsau (CH); GURTNER, Thomas, CH-3425 Koppigen (CH)
(86) Internationale Anmeldenummer: PCT/CH2001/000469
(87) Internationale Veröffentlichungsnummer: WO 2002/026297

(56) Entgegenhaltungen:
- EP-A- 0 962 229
- DE-C- 19 900 792

## Beschreibung

Die Erfindung betrifft eine Vorrichtung zur Verabreichung eines injizierbaren Produkts nach dem Oberbegriff von Anspruch 1.

Ein Injektionsgerät, wie die Erfindung es auch betrifft, ist aus der WO 97/36625 und der DE 199 00 792 C1 bekannt. Das Injektionsgerät umfasst ein Gehäuse, ein Produktreservoir mit einem darin verschiebbar aufgenommenen Kolben, bei dessen Verschiebung in eine Vorschubrichtung Produkt aus dem Reservoir verdrängt wird, eine Antriebseinrichtung und eine Dosiereinrichtung.

Die Antriebseinrichtung umfasst ein Antriebsglied, das in die Vorschubeinrichtung verschiebbar ist, und ein Abtriebsglied, das an einer Verschiebung gegen die Vorschubrichtung gehindert ist, bei einer Verschiebung des Antriebsglieds in Vorschubrichtung jedoch von dem Antriebsglied mitgenommen wird und dabei den Kolben in Vorschubrichtung schiebt, so dass Produkt aus dem Reservoir verdrängt wird. Die ausschüttbare Produktdosis wird mittels einer Dosiereinrichtung eingestellt.

Die Dosiereinrichtung umfasst das Antriebsglied und das Dosierglied zur Einstellung der distalen Endposition des Antriebsglieds. Das Dosierglied ist um die Verschiebeachse des Antriebsglieds verdrehbar in dem Gehäuse gelagert. Es weist vorzugsweise einen um diese Verschiebeachse spiralig umlaufenden Dosieranschlag mit vorzugsweise einem kontinuierlichen Verlauf mit einer konstanten Steigung relativ zur Verschiebeachse des Antriebsglieds auf, an den das Antriebsglied bei einer Verschiebung in die distale Position anschlägt, d.h. die Drehwinkelposition des Dosierglieds bestimmt die distale Position des Antriebsglieds.

Die ausschüttbare Produktdosis wird durch Verdrehen des Dosierglieds vorzugsweise in diskreten Schritten gewählt. Hierfür verrastet das Dosierglied in regelmäßig zwischen dem Gehäuse und dem Dosierglied gebildeten Drehwinkelrastpositionen. Eine Verdrehung des Dosierglieds zwischen zwei benachbarten Rastpositionen entspricht einer einstellbaren, kleinsten Produktdosis.

Die Erfindung hat es sich zur Aufgabe gemacht, eine Vorrichtung zur Verabreichung eines injizierbaren Produkts, insbesondere der vorgenannten Art, zu schaffen, die für einen Anwender eine leichte Produktdosierung ermöglicht, wobei die Feinheit und Exaktheit der Dosierung der vorbekannten Vorrichtung zumindest beibehalten werden soll. Insbesondere soll für eine leichte Verdrehbarkeit des Dosierglieds gesorgt werden.

Diese Aufgabe wird durch den Gegenstand von Anspruch 1 gelöst, indem eine Lagerfläche zwischen dem Dosierglied und dem Gehäuse geschmiert wird und an der einen Lagerfläche wenigstens ein Schmiermittelreservoir ausgebildet ist.

Durch die Schmierung der Lagerfläche wird die Reibung zwischen Dosierglied und Gehäuse verringert. Für einen Anwender wird das für eine Dosierung durchzuführende Bewegen, vorzugsweise Verdrehen, des Dosierglieds erleichtert. Durch die Anordnung wenigstens eines Schmiermittelreservoirs kann genügend Schmiermittel zur Verfügung gestellt werden, so dass während der gesamten Gebrauchsdauer der Vorrichtung keine nachträgliche Schmiermittelzufuhr notwendig ist. Es wird für eine Lebenszeitschmierung gesorgt. Dies vereinfacht den Gebrauch der Vorrichtung.

Das Schmiermittelreservoir ist vorzugsweise in unmittelbarer Nähe an der Lagerfläche ausgebildet. Es wird vorzugsweise von einem Hohlraum im Dosierglied oder im Gehäuse gebildet.

Vorzugsweise wird das Dosierglied um die Verschiebeachse des Antriebsglieds drehbar und axialverschiebesicher im Gehäuse gelagert. Besonders bevorzugt ist zwischen dem Dosierglied und dem Gehäuse kein Längsspiel vorhanden. Dadurch wird für eine genaue Dosierung der Vorrichtung gesorgt. In der Lagerfläche zwischen dem Dosierglied und dem Gehäuse herrscht dadurch eine hohe Flächenpressung. Diese hohe Flächenpressung ergibt ein hohes Drehmoment, welches jedoch wirksam durch eine Schmierung reduziert wird.

Besonders bevorzugt wird das Dosierglied über einen Ringwulst axial verschiebesicher am Gehäuse gelagert. Der Ringwulst ist vorzugsweise an derjenigen Mantelfläche des Dosierglieds angeordnet, die mit dem Gehäuse in Berührung ist, wobei der Ringwulst in eine Ausnehmung in der Mantelfläche des Gehäuses hineinragt. Zwischen dem Dosierglied und dem Gehäuse wird somit eine Hinterschneidung erzeugt. Grundsätzlich könnte der Ringwulst jedoch auch am Gehäuse ausgebildet werden. Eine in einem Winkel zur Verschiebeachse stehende Fläche des Ringwulstes bildet eine Lagerfläche für die axiale Lagerung des Dosierglieds. Vorzugsweise wird diese Fläche geschmiert und an ihr wenigstens ein Schmiermittelreservoir ausgebildet. Das Schmiermittelreservoir wird derart angeordnet, dass das sich darin befindliche Schmiermittel bei einer Drehung des Dosierglieds mit der Lagerfläche in Kontakt kommt.

Vorzugsweise wird auch eine zweite Lagerfläche für die axiale Lagerung des Dosierglieds geschmiert und an ihr wenigstens ein Schmiermittelreservoir ausgebildet. Diese zweite Lagerfläche kann von einer weiteren Fläche des Ringwulstes oder von einer anderen Fläche des Dosierglieds oder des Gehäuses gebildet werden.

Vorzugsweise ist der Ringwulst am Dosierglied ausgebildet und in einer Ausnehmung im Gehäuse axial gehalten. Das Dosierglied ist besonders bevorzugt innerhalb des Gehäuses angeordnet, so dass der Ringwulst radial von der Außenmantelfläche des Dosierglieds in eine Ringnut, welche an der Innenmantelfläche des Gehäuses ausgebildet ist, abragt.

Das Schmiermittelreservoir wird vorzugsweise durch eine Abflachung des Ringwulstes gebildet. Besonders bevorzugt ist der Ringwulst an der Stelle, an der das Schmiermittelreservoir ausgebildet wird, unterbrochen. Durch die Unterbrechung des Ringwulstes entsteht ein Hohlraum zwischen dem Dosierglied und der Ringnut im Gehäuse. In diesen Hohlraum kann Schmiermittel eingelagert werden. Ein Vorteil des Schmiermittelreservoirs besteht darin, dass bei der Montage des Dosierglieds in das Gehäuse das Schmiermittel zwar von der Lagerfläche weggedrückt wird, bei einem Verdrehen die Lagerfläche jedoch über ein Schmiermittelreservoir läuft, so dass eine ständige Nachschmierung gewährleistet ist. Das Schmiermittel bleibt auch nach der Montage an der gewünschten Stelle.

Vorzugsweise werden mehrere Schmiermittelreservoirs gleichmäßig über den Umfang des Dosierglieds oder des Gehäuses verteilt angeordnet. Dadurch wird eine bessere Schmierung der Lagerfläche erreicht. Besonders bevorzugt werden vier Schmiermittelreservoirs vorgesehen.

Als Schmiermittels wird vorzugsweise Fett besonders bevorzugt Molykote-Fett verwendet. Die Schmiermittelreservoirs werden als Fettaschen ausgebildet.

Ein bevorzugtes Ausführungsbeispiel der Erfindung wird nachfolgend anhand von Figuren erläutert. Es zeigen:
- Fig. 1: eine Injektionsvorrichtung mit einer Dosiereinrichtung in einem Längsschnitt und
- Fig. 2: ein Dosierglied mit einem erfindungsgemäßen Schmiermittelreservoir gemäß Detail A von Fig. 1.

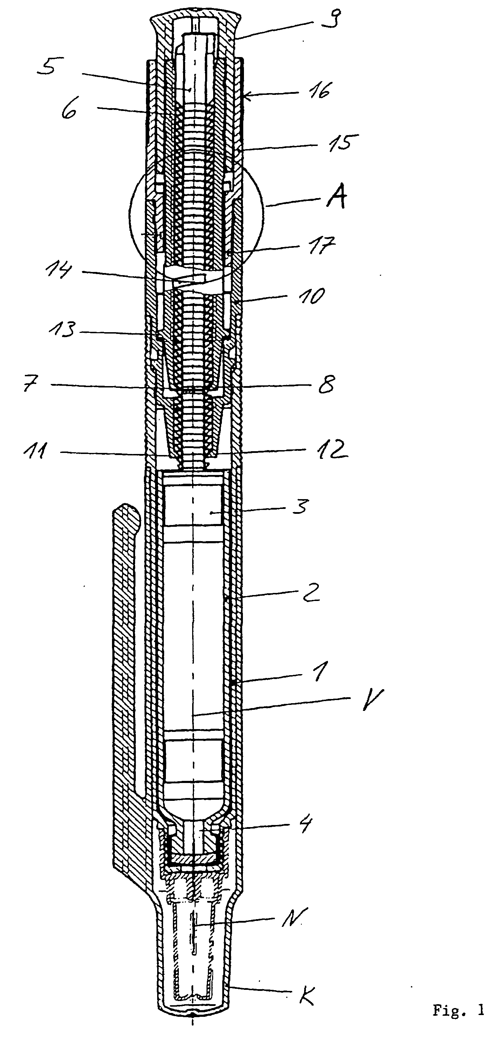

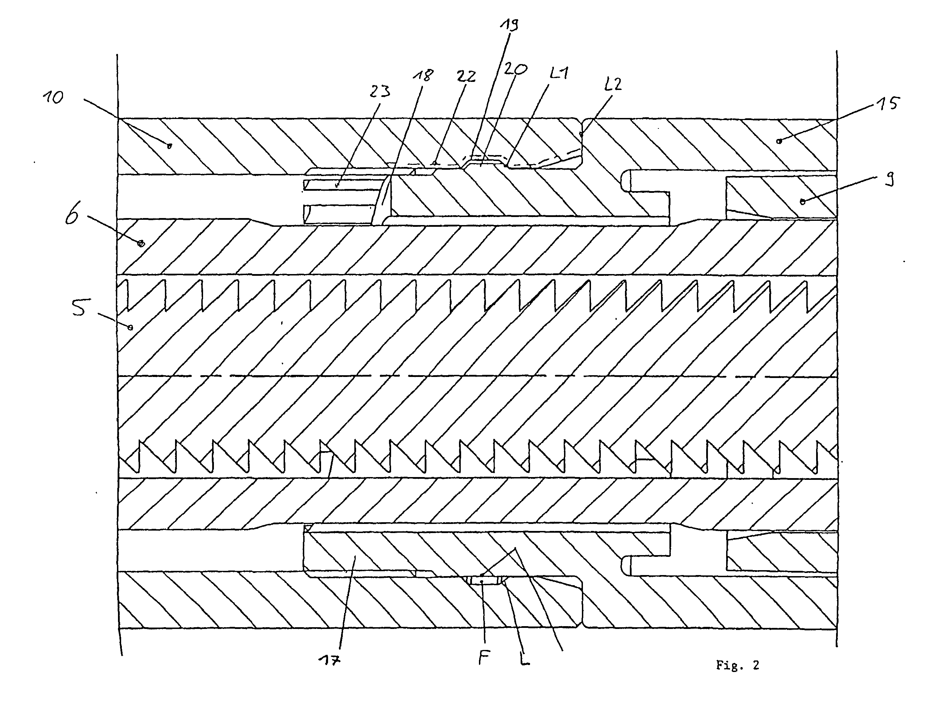

Figur 1 zeigt ein Injektionsgerät, im Ausführungsbeispiel ein Injektionspen, in einem Längsschnitt. Die Figur 2 zeigt Detail A von Fig. 1.

Das Injektionsgerät weist ein Gehäuse mit einer vorderen Gehäusehülse 1 und einer damit fest verbundenen hinteren Gehäusehülse 10 auf. Die vordere Gehäusehülse dient als Aufnahme für eine Ampulle 2. In der Ampulle 2 ist ein flüssiges Produkt in Form einer Wirkstofflösung, beispielsweise Insulin, enthalten. Ferner ist in der Ampulle 2 ein Kolben 3 aufgenommen. Durch Verschiebung des Kolbens 3 in Vorschubrichtung auf einen Ampullenauslass 4 zu wird das Produkt aus der Ampulle 2 durch deren Auslass 4 hindurch verdrängt und durch eine Injektionsnadel n, auch bei 31G und grösser ausgeschüttet. Die vordere Gehäusehülse 1 ist durch eine Kappe K geschützt. Die Nadel N ist durch eine Nadelkappe nochmals geschützt.

Die Verschiebung des Kolbens 3 in Vorschubrichtung wird durch eine Antriebseinrichtung bewirkt, die in der hinteren Gehäusehülse 10 aufgenommen ist. Die Antriebseinrichtung umfasst als Abtriebsglied eine Zahnstange 5, die unmittelbar auf den Kolben 3 wirkt, und ein Antriebsglied 6. Das Antriebsglied 6 ist in der hinteren Gehäusehülse 10 in und gegen die Vorschubrichtung des Kolbens 3 entlang einer Verschiebeachse V geradverschiebbar gelagert. Ein Deckel 9, der mit dem Antriebsglied 6 verschiebe- und frei verdrehbar verbunden ist, ragt aus dem Gehäuse nach hinten heraus.

Ein als Hülsenkörper ausgebildetes Dosierglied ist mit der hinteren Gehäusehülse 10 verschiebegesichert, jedoch um die gemeinsame Längsachse, die mit der Verschiebeachse V zusammenfällt, verdrehbar verbunden. Das Dosierglied 15 ragt mit einem vorderen Hülsenteil 17 in die hintere Gehäusehülse 10. Sein hinterer Hülsenteil ragt aus der hinteren Gehäusehülse 10 heraus. Das hintere Hülsenteil des Dosierglieds 15 ist mit einer Profilierung 16 versehen, um das Dosierglied 15 griffsicher manuell verdrehen zu können.

Wie am besten aus der Zusammenschau der Figuren 1 und 2 zu erkennen ist dient zur verschiebesicheren Befestigung des Dosierglieds 15 ein an dem vorderen Hülsenteil 17 ausgebildeter Ringwulst 20, der in einer an dem Innenmantel der hinteren Gehäusehülse 10 umlaufenden Ausnehmung 19 eingeschnappt ist. Eine Fläche L1 des Ringwulstes 20 kommt mit einer hinterschnittenen Fläche der Ausnehmung 19 der hinteren Gehäusehülse 10 in Kontakt. Eine zweite Lagerfläche L2 wird zwischen dem hinteren Ende der hinteren Gehäusehülse 10 und einem umlaufenden, radial abstehenden Vorsprung des Dosierglieds 15 gebildet. Das Dosierglied 15 wird zwischen den Lagerflächen L1 und L2 axial verschiebesicher im Gehäuse gelagert.

Vor der Profilierung 16 trägt das Dosierglied 15 um seine äußere Mantelfläche umlaufend eine gut sichtbare Dosisskala, die auf festgelegte Drehwinkelpositionen, in denen das Dosierglied 15 gegen die hintere Gehäusehülse 10 verrastet, abgestimmt ist. Der Rastmechanismus zwischen dem Dosierglied 15 und der hinteren Gehäusehülse 10 wird durch Erhebungen an dem Außenmantel des vorderen Hülsenteils 17 des Dosierglieds 15 und Vertiefungen 23 sind in dem Innmantel der hinteren Gehäusehülse 10 gebildet. Die Vertiefungen 23 sind in gleichen Winkelabständen nebeneinander auf gleicher Höhe umlaufend an dem Innenmantel der hinteren Gehäusehülse 10 angeordnet. In den fixen Drehwinkelrastpositionen des Dosierglieds 15 werden die mehreren Erhebungen exakt in den jeweils gegenüberliegenden Vertiefungen in dem Innenmantel der hinteren Gehäusehülse 10 aufgenommen.

Im komplett montierten Zustand des Injektionsgerätes, wie es in Figur 1 dargestellt ist, wird das Dosierglied 15 von dem Antriebsglied 6 durchragt. Das Dosierglied 15 umgibt einen distalen Teil des Antriebsglieds 6 und auch des Abtriebglieds 5 konzentrisch. Der Deckel 9 ragt mit einem Hülsenteil in einem zwischen dem Antriebsglied 6 und dem Dosierglied 15 gebildeten Ringspalt hinein. Auch der Deckel 9 trägt in seinem aus dem Dosierglied 15 herausragenden Mantelbereich eine Markierung, die im Zusammenwirken mit der Markierung des Dosierglieds 15 auch nach mehreren vollen Drehungen des Dosierglieds 15 die genaue Bestimmung der aus der Ampulle 2 insgesamt verabreichten Produktmenge ermöglicht.

Durch Verdrehen des Dosierglieds 15 wird die in Vorschubrichtung von dem Antriebsglied 6 und der Zahnstange 5 maximal zurücklegbare Dosisweglänge eingestellt und damit auch die bei einer Injektion, auch bei 31 G und grösser maximal ausschüttbare Produktdosis.

Je geringer das axiale Spiel des Dosierglieds 15 in der hinteren Gehäusehülse 10 ist, desto genauer ist die Dosierung der Vorrichtung. Vorzugsweise ist das Dosierglied 15 ohne Spiel in der hinteren Gehäusehülse 10 aufgenommen. Damit steigt jedoch die Flächenpressung in den Lagerflächen L1, L2, so dass vom Verwender ein höheres Drehmoment zum Einstellen der Dosis notwendig wäre. Durch die Schmierung wenigstens einer der Gleitlagerflächen L1, L2 kann dieses Drehmoment reduziert werden. Da bei einer hohen Flächenpressung das Schmiermittel von den Lagerflächen L1, L2 bei der Montage oder im Betrieb verdrängt würde, werden Schmiermittelreservoirs F vorgesehen, die einen Kontakt zwischen der Lagerfläche L1 oder L2 und dem Schmiermittel herstellen.

Ein Schmiermittelreservoir F wird durch eine abschnittsweise Unterbrechung des Ringwulstes 20 gebildet. Dadurch ergibt sich ein abschnittsweiser Zwischenraum zwischen dem Dosierglied 15 und der Gehäusehülse 10. Dieser Zwischenraum kann mit Schmiermittel, insbesondere Fett, gefüllt werden und dient als Lebensdauerschmierung für die Lagerfläche L1. Vorzugsweise werden mehrere Schmiermittelreservoirs F gleichmäßig über den Umfang verteilt angeordnet. Je mehr Schmiermittelreservoirs F vorgesehen werden, desto öfter überstreicht die Lagerfläche L1 bei einer Drehung ein Schmiermittelreservoir F. Der Schnitt gemäß Fig. 2 verläuft im oberen Berreich der Zeichnung durch den Ringwulst 20 und im unteren Bereich der Zeichnung durch ein Schmiermittelreservoir F.

Nicht in Figur 2 dargestellt ist eine Weiterbildung eines Schmiermittelreservoirs F, bei dem das Volumen dadurch erhöht wird, dass im Bereich der Unterbrechung des Ringwulstes 20 zusätzlich eine Vertiefung in dem Dosierglied 15 vorgesehen ist. Nicht in Figur 2 dargestellt ist ebenfalls die Variante, bei der der Ringwulst 20 an der hinteren Gehäusehülse 10 angeordnet ist. Die Ausbildung der Schmiermittelreservoirs F erfolgt dabei in analoger Weise. Analog zu den Schmiermittekeservoirs im Ringwulst 20 können auch Schmiermittelreservoirs F in der Lagerfläche L2 vorgesehen werden. Vorzugsweise werden sie durch Ausnehmungen in der radialen Stirnfläche des Dosierglieds 15 oder der hinteren Endfläche der hinteren Gehäusehülse 10 gebildet. Vorzugsweise werden Schmiermittelreservoirs F nur in der Lagerfläche L1 ausgebildet. Sie können jedoch auch in der Lagerfläche L2 ausgebildet werden. Besonders bevorzugt befinden sich in beiden Lagerflächen L1 und L2 Schmiermittelreservoirs F.

Vor der Montage des Dosierglieds 15 wird der in Figur 2 gestrichelt eingezeichnete Bereich 22 der hinteren Gehäusehülse 10 mit Schmiermittel versehen. Wird zur Montage das Dosierglied 15 in die hintere Gehäusehülse 10 geschoben, bleibt in den Bereichen der Schmiermittelreservoirs F das Schmiermittel zurück und sorgt bei einer Verdrehung des Dosierglieds 15 für eine Schmierung der Lagerflächen L.

Die Dosierung erfolgt in einer in Bezug auf die Vorschubrichtung vordersten, proximalen Endposition des Antriebsglieds 6, in der ein von der äußeren Mantelfläche des Antriebsglieds 6 radial abstehender Anschlagnocken oder -kragen 13 an einem durch die hintere Gehäusehülse 10 gebildeten Anschlag anliegt. In dieser proximalen Endposition des Antriebsglieds 6 wird das Dosierglied 15 um die Verschiebeachse V relativ zur hinteren Gehäusehülse 10 verdreht, bis es die gewünschte Dosier bzw. Drehwinkelrastposition erreicht hat. In dieser Dosierposition verbleibt zwischen einem ebenfalls von der äußeren Mantelfläche des Antriebsglieds 6 abragenden weiteren Kragen bzw. Nocken, der einen Dosieranschlag bildet und daher im weiteren Dosiemocken 14 genannt wird, und der diesem Dosiernocken 14 gegenüberliegenden, proximalen Stirnseite 18 des Dosierglieds 15 ein lichter Dosierabstand. Um den Dosierabstand kann das Antriebsglied 6 relativ zur hinteren Gehäusehülse 10 und damit auch relativ zum Kolben 3 gegen die Vorschubrichtung zurückgezogen werden. Das Zurückziehen erfolgt manuell durch Ziehen an dem Deckel 9. Der Dosierabstand ist gleich der Dosisweglänge bei der nachfolgenden Verabreichung.

Bei einer Zurückverschiebung bzw. Zurückziehen des Antriebsgliedes 6 verbleibt die Zahnstange 5 in ihrer bei dem Dosiervorgang eingenommenen Verschiebelage relativ zum Gehäuse. Sie wird durch an der hinteren Gehäusehülse 10 ausgebildete Sperrmittel 11 und 12 gegen eine Verschiebung gegen die Vorschubrichtung gesichert. Die Sperrmittel 11 und 12 sind Rastnocken, die je an einem der vorderen Ende einer elastisch nachgiebigen Zunge ausgebildet sind und von ihrer Zunge radial nach innen auf die Zahnstange 5 zu ragen. Die Sperrmittel 11 und 12 wirken je mit einer ihnen zugewandten Zahnreihe der Zahnstange 5 zusammen, derart, dass sie eine Verschiebung der Zahnstange 5 in Vorschubrichtung zulassen und eine Verschiebung gegen die Vorschubrichtung zulassen und eine Verschiebung gegen die Vorschubrichtung durch formschlüssigen Sperreingriff verhindern. Nach dem Zurückziehen des Antriebsglieds 6 kann es um die Dosisweglänge in Vorschubrichtung verschoben werden, wobei es die Zahnstange 5 und den Kolben 3 mitnimmt, so dass eine dosierte Menge des injizierbaren Produkts aus dem Reservoir 2 verdrängt wird.

## Patentansprüche

1. Vorrichtung zur dosierten Verabreichung eines injizierbaren Produkts, die Vorrichtung umfassend:
a) ein Gehäuse (1), mit einem Reservoir (2) für das Produkt,
b) einen Kolben (3), der bei einer Verschiebung in eine Vorschubrichtung auf einen Reservoirauslass (4) zu Produkt aus dem Reservoir (2) verdrängt,
c) ein Antriebsglied (6), welches entlang einer Verschiebeachse (V) verschiebbar ist und dabei den Kolben (3) in Vorschubrichtung schiebt,
d) ein Dosierglied (15), das zur Einstellung einer bei einer Verabreichung ausschüttbaren Produktdosis um die Verschiebeachse (V) des Antriebsglieds (6) gleitend bewegbar, vorzugsweise drehbar, im Gehäuse (1) gelagert ist,
**dadurch gekennzeichnet, dass**
e) eine Lagerfläche (L) zwischen dem Dosierglied (15) und dem Gehäuse (1) bei der Dosierung geschmiert wird und
f) an der einen Lagerfläche (L) wenigstens ein Schmiermittelreservoir (F) ausgebildet ist.

2. Vorrichtung nach dem vorhergehenden Anspruch, **dadurch gekennzeichnet, dass** das Dosierglied (15) über einen Ringwulst (20) axial verschiebesicher am Gehäuse (1) gelagert ist und die Lagerfläche (L) durch wenigstens eine Fläche des Ringwulstes (20) gebildet wird.

3. Vorrichtung nach dem vorhergehenden Anspruch, **dadurch gekennzeichnet, dass** der Ringwulst (20) am Dosierglied (15) ausgebildet ist und in einer Ringnut im Gehäuse (1) axial gehalten wird.

4. Vorrichtung nach einem der beiden vorhergehenden Ansprüche, **dadurch gekennzeichnet, dass** das wenigstens ein Schmiermittelreservoir (F) durch eine Abflachung des Ringwulstes (20) gebildet wird.

## Claims

1. A device for the dosaged administration of an injectable product, the device including:
a) a casing (1) having a reservoir (2) for the product,
b) a piston (3) which upon displacement in a feed direction towards a reservoir outlet (4) expels product from the reservoir (2),
c) a drive member (6) which is displaceable along a displacement axis (V) and in such displacement displaces the piston (3) in the feed direction, and
d) a dosing member (15) which for setting a product dose which can be expelled in an administration procedure is mounted in the casing (1) slidingly movable, preferably rotatable, about the displacement axis (V) of the drive member (6),
**characterised in that**
e) a bearing surface (L) between the dosing member (15) and the casing (1) is lubricated in the dosing operation, and
f) at least one lubricant reservoir (F) is provided at the one bearing surface (L).

2. A device according to the preceding claim **characterised in that** the dosing member (15) is mounted on the casing (1) axially non-displaceably by way of an annular ridge (20) and the bearing surface (L) is formed by at least one surface of the annular ridge (20).

3. A device according to the preceding claim **characterised in that** the annular ridge (20) is provided on the dosing member (15) and is held axially in an annular groove in the casing (1).

4. A device according to one of the two preceding claims **characterised in that** the at least one lubricant reservoir (F) is formed by a flattening of the annular ridge (20).

## Revendications

1. Dispositif permettant d'administrer une dose d'un produit injectable, le dispositif comprenant :
a) un boîtier (1) comportant un réservoir (2) pour le produit,
b) un piston (3) qui, lors d'une poussée exercée dans une direction d'avance sur une sortie de réservoir (4), repousse du produit hors du réservoir (2),
c) un membre d'entraînement (6) qui peut se déplacer sur un axe de déplacement (V) tout en poussant le piston (3) dans la direction d'avance,
d) un membre doseur (15) qui est posé dans le boîtier (1) de manière à pouvoir glisser, de préférence tourner, autour de l'axe de déplacement (V) du membre d'entraînement, pour ajuster une dose de produit pouvant être déversée lors d'une administration,
**caractérisé en ce que**
e) une surface d'appui (L) entre le membre doseur (15) et le boîtier (1) est lubrifiée lors du dosage et
f) au moins un réservoir à lubrifiant (F) se trouve sur une surface d'appui (L).

2. Dispositif selon la revendication précédente, **caractérisé en ce que** le membre doseur (15) est disposé de manière à ne pas pouvoir bouger axialement dans le boîtier (1) grâce à un bourrelet annulaire (20) et la surface d'appui (L) est formée d'au moins une face du bourrelet annulaire (20).

3. Dispositif selon la revendication précédente, **caractérisé en ce que** le bourrelet annulaire (20) est formé sur le membre doseur (15) et est maintenu axialement dans une rainure annulaire du boîtier (1).

4. Dispositif selon l'une quelconque des deux revendications précédentes, **caractérisé en ce qu'**au moins un réservoir à lubrifiant (F) est formé par un aplatissement du bourrelet annulaire (20).
